# FASCICULE DE BREVET EUROPEEN

(11) **EP 3 658 055 B1**
(45) Date de publication et mention de la délivrance du brevet: **03.07.2024**
(21) Numéro de dépôt: 18782476.8
(22) Date de dépôt: 24.07.2018
(51) Int. Cl.: A61B 18/24, A61B 17/11

(54) **SONDE A FIBRE OPTIQUE COMPRENANT UN BROUILLEUR DE MODE**
FASEROPTISCHE SONDE MIT MODUSVERSCHLÜSSLER
FIBER OPTIC PROBE COMPRISING A MODE SCRAMBLER

(30) Priorité: 24.07.2017 FR 1757015
(43) Date de publication de la demande: 03.06.2020
(73) Titulaire: Sedi-ati Fibres Optiques, 91080 Courcouronnes (FR)
(72) Inventeur: MALAVIEILLE, Jean-Michel, 91540 Mennecy (FR); LASKRI, Samir, 91130 Ris Orangis (FR)
(74) Mandataire: IP Trust
(86) Numéro de dépôt international: PCT/FR2018/051891
(87) Numéro de publication internationale: WO 2019/020926

(56) Documents cités:
- EP-A1- 0 297 190
- US-A- 4 625 724
- US-A1- 2010 179 525
- US-A1- 2016 184 022

## Description

### DOMAINE TECHNIQUE DE L'INVENTION

L'invention telle que définie dans la revendication 1 ci-dessous concerne une sonde comprenant une fibre optique comportant, en extrémité distale, une tête d'émission, de forme conique, pour l'émission annulaire du rayonnement optique en provenance d'une source optique.

La sonde est destinée notamment, mais non exclusivement, pour le traitement thermique endo-veineux, et en particulier la rétractation par sclérose des conduits veineux ou de tout autre conduit vasculaire.

### ETAT DE LA TECHNIQUE

De manière connue, la rétractation par sclérose de conduits vasculaires est réalisée par application de rayons laser au niveau de la paroi du conduit à rétracter par sclérose. Elle est réalisée classiquement à l'aide d'une sonde présentant en extrémité distale une pointe conique arrangée pour assurer une réflexion totale des rayons laser puis à permettre aux rayons laser réfléchis d'émaner de la pointe, en passant par la paroi opposée à la paroi réfléchissant lesdits rayons de manière à former un faisceau annulaire de rayons laser appliquée à la paroi intérieure du conduit vasculaire à rétracter par sclérose. La rétractation par sclérose est réalisée en insérant la sonde dans la partie du conduit puis en appliquant les rayons laser émanant de la tête projetés radialement sur la paroi. Un exemple d'une telle sonde est décrite dans le brevet US4625724. Les documents US 2016/184022 et EP 0 297 190 décrivent d autres exemples de sondes connues.

Les sondes de l'art antérieur restent cependant limitées en terme d'ouverture numérique du rayonnement émanant de leurs têtes d'émission. Par ailleurs, lors de leur fonctionnement, les têtes d'émission subissent des échauffements importants. En réaction à l'échauffement de la tête, le sang situé au niveau de la zone d'échauffement se coagule et se colle sur la capsule de protection de la tête dont les sondes sont classiquement équipées. Cela cause invariablement une détérioration de la capsule avec un risque parfois que celles-ci ne se cassent ou se désolidarisent de la tête de diffusion de la sonde. Il s'ensuit un risque pour le patient.

L'invention vise à remédier à ces problèmes en proposant une sonde permettant conjointement de limiter l'échauffement de la tête d'émission et d'homogénéiser le rayonnement optique en sortie de la tête d'émission de la sonde.

L'invention a également pour but de proposer une sonde pour le traitement thermique endo-veineux avec ouverture numérique améliorée.

### OBJET DE L'INVENTION

A cet effet, et selon un premier aspect, l'invention propose une sonde comprenant une fibre optique comportant, en extrémité distale, une tête d'émission, de forme conique, pour l'émission d'un rayonnement optique en provenance d'une source optique, la sonde étant remarquable en ce que la fibre optique comporte un coeur en silice entourée d'une gaine optique en polymère et en ce qu'elle comporte une structure tubulaire, de préférence métallique, cintrée de façon hélicoïdale et entourant la fibre optique. Plus spécifiquement, la fibre optique présente une portion longitudinale disposée à l'intérieur d'un tube cintré de sorte à conférer à la portion longitudinale de ladite fibre une courbure hélicoïdale. Le tube est ainsi traversé par la fibre optique. Cet arrangement est obtenu par exemple en introduisant la fibre optique dans un tube sensiblement droit puis en cintrant ce dernier. Le tube est constitué d'un matériau ductile, de préférence métallique.

Le tube hélicoïdal disposé ainsi au sein de la sonde constitue un brouilleur de mode. Par ailleurs, la réalisation en silice/polymère de la fibre optique offre une ouverture supérieure aux fibres classiques Silice/Silice.

En combinant un tube hélicoïdal avec une fibre en silice / polymère, on obtient un rayonnement émanant de la sonde qui est homogène et non radial et qui offre une ouverture numérique améliorée. Par non radial, on entend un rayonnement qui est projeté en aval de la tête de diffusion. On désigne l'aval par rapport à la tête de diffusion suivant le sens d'émission du rayonnement optique. L'avantage du rayonnement obtenu avec la sonde est de limiter les échauffements locaux au niveau de la tête d'émission en éloignant la projection du rayonnement optique lumineux sur la paroi de la tête d'émission de la fibre, ainsi qu'en répartissant de manière homogène le rayon lumineux. Cela élimine ainsi les risques de détérioration de la capsule de protection ménagée au niveau de la tête. Cela garantit de plus un fonctionnement de la sonde constant durant toute la durée de l'opération de la rétractation par sclérose sans risque de détérioration ou de désagrégement de la capsule. Le risque pour le patient de subir des complications inhérentes à l'utilisation de la sonde est ainsi fortement réduit au regard de ceux encourus en utilisant les sondes de l'art antérieur.

Avantageusement, le tube présente :
- un pas d'enroulement compris entre 20 et 40 millimètres, et de préférence de 30 millimètres et/ou
- un diamètre d'enroulement comprise entre 6 et 10 millimètres, et de préférence 8 millimètres et/ou
- une longueur, en état déployée, comprise entre 80 et 120 millimètres, et de préférence de 100 millimètres.

Avantageusement, le tube est situé à une distance d'un connecteur d'injection reliant la fibre optique à une source optique comprise entre 50 centimètres et 100 centimètres. Cette distance est donnée pour une longueur classique de fibre optique comprise entre 2,5 mètres à 3 mètres.

Avantageusement, la tête d'émission présente une forme conique avec un angle au sommet compris entre 55 et 59 degrés, et de préférence 57 degrés.

Selon un mode de réalisation particulier, la tête de diffusion a la forme d'une ogive ayant une génératrice courbe, afin de modifier l'ouverture numérique du faisceau.

Avantageusement, la tête d'émission est pourvue d'une capsule de protection transparente au rayonnement optique diffusé par la tête d'émission. La capsule est réalisée de préférence dans le même matériau que le coeur de la fibre optique, à savoir la silice.

La sonde précédemment décrite est destinée à être utilisée notamment mais non exclusivement dans le cadre d'un traitement endo-veineux.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique d'une sonde selon un premier mode de réalisation de l'invention ;
- la figure 1A représente une vue en coupe longitudinale partielle de la sonde de la figure 1 ;
- la figure 2 représente le façonnage du brouilleur de mode mis en oeuvre dans la sonde illustrée sur la figure 1 ;
- la figure 3 représente une vue de détail de la tête de diffusion de la sonde positionnée dans un conduit de la figure 1 et montrant le chemin du rayonnement optique en sortie de la tête de diffusion ;
- la figure 4 représente une vue schématique de la tête de diffusion d'une sonde selon un autre mode de réalisation de l'invention.

Pour plus de clarté, les éléments identiques ou similaires des différents modes de réalisation sont repérés par des signes de référence identiques sur l'ensemble des figures.

### DESCRIPTION DETAILLEE DES FIGURES

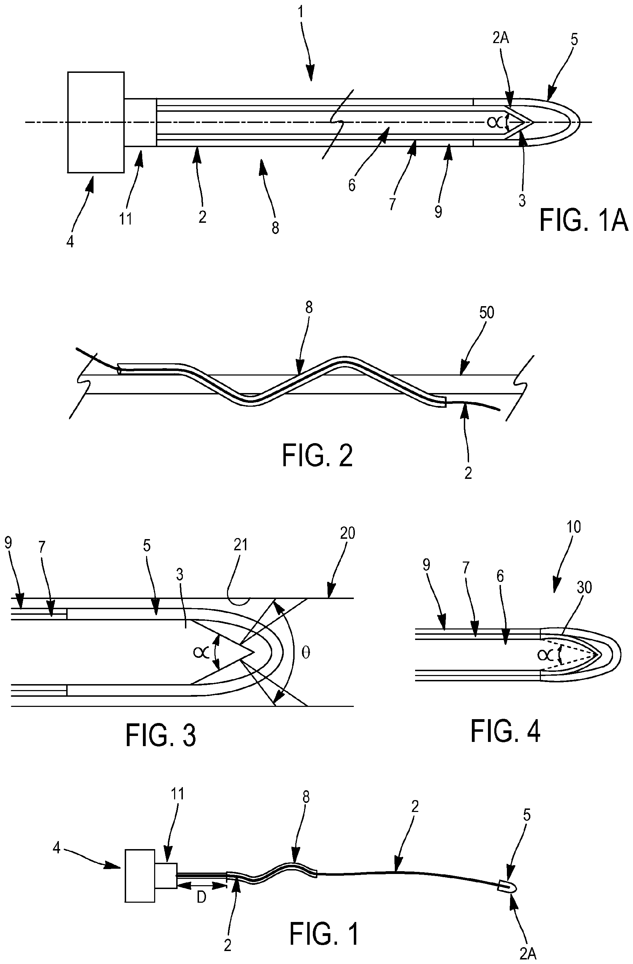

La figure 1 représente une vue schématique d'une sonde 1 médicale selon l'invention destinée notamment au traitement thermique endo-veineux.

La sonde 1 médicale comporte une fibre optique 2 comprenant, de manière connue, un coeur 6 revêtue d'une gaine optique 7 elle-même recouverte d'une gaine de protection 9 mécanique (figure 1A).

La fibre optique 2 présente, en extrémité distale 20, une tête d'émission 3 arrangée pour émettre un rayonnement optique en provenance d'une source optique 4, tel qu'un laser et guidé dans le coeur 6 depuis la source laser jusqu'à la tête de d'émission 3. La tête de d'émission 3, et plus spécifiquement le coeur optique 6 de la tête d'émission, présente une forme conique obtenue par exemple par polissage.

La tête d'émission 3, dénudée de la gaine de protection 9, est pourvue d'une capsule de protection 5 transparente au rayonnement optique émanant de la tête de d'émission. L'assemblage de la capsule de protection 5 à la fibre optique 2 est réalisé par collage.

Dans la sonde 1 selon l'invention, le coeur 6 de la fibre optique 2 est réalisé en silice, et plus particulièrement en silice pure, tandis que la gaine optique 7 est réalisé en polymère dur. La capsule de protection 5 est réalisée de préférence dans le même matériau que le coeur 6, en l'espèce en silice. La gaine de protection 9 est quant à elle réalisée en polyamide.

La réalisation du coeur 6 et de la gaine optique 7 de la fibre optique 2 en ces matériaux permet d'améliorer l'ouverture numérique. Plus particulièrement, l'ouverture numérique obtenue avec une fibre optique silice / polymère est augmentée d'environ 50% par rapport à celle obtenue avec les fibres optiques de l'art antérieur mise en oeuvre dans des sondes médicales et réalisées classiquement en silice/silice.

Afin de maximiser davantage l'ouverture numérique, la tête de diffusion 3 présente une forme conique avec un angle au sommet α compris entre 55 et 59 degrés Selon une configuration optimale, la tête d'émission 3 est arrangée pour présenter un angle au sommet α de 57 degrés.

En mettant en oeuvre une fibre optique en silice/polymère présentant une tête d'émission conique avec un angle au sommet α compris entre 55 et 59 degrés préférentiellement de degrés, et préférentiellement 57 degrés, le rayonnement optique, en sortie de la tête d'émission 2 et de la capsule de protection 5 et projeté sur la paroi 21 du conduit 20 veineux dans lequel la tête de diffusion est placée, est émis suivant un angle θ de 120 degrés et non plus radialement comme avec les sondes de l'art antérieur, ce qui permet d'atteindre une surface de la paroi 21 veineuse plus important. La figure 3 montre le chemin du rayonnement optique obtenu en sortie d'une telle sonde.

La sonde 1 comporte une structure, de préférence métallique, entourant la fibre optique 2 et maintenant celle-ci dans une position hélicoïdale. Plus particulièrement, la structure est un tube 8 de forme hélicoïdale traversé par la fibre optique 2 .

La figure 2 montre un mode de réalisation de la fibre optique équipée d'un tel tube hélicoïdal. La fibre optique 2 est enfilée dans un tube métallique 8 lui-même enroulé de façon hélicoïdale autour d'un axe 50 (mandrin) servant à façonner la forme hélicoïdale du tube autour de la fibre optique. La présence de cette structure permet d'homogénéiser la propagation de la lumière dans la fibre optique, en assurant une meilleure répartition de la lumière issue de tête d'émission de façon beaucoup plus performante que celle offerte par les sondes de l'art antérieur et ainsi d'éviter les points chauds. Le tube hélicoïdal 8 ainsi arrangé avec la fibre optique 2 constitue un brouilleur de mode.

Selon une configuration préférée illustrée, le tube hélicoïdal 8 présente un pas sensiblement de 30 millimètres et une longueur, à l'état déployé, de l'ordre de 100 millimètres. D'autres dimensionnements peuvent bien entendu être prévus sans sortir du champ de l'invention. Cependant, afin d'assurer un homogénéité suffisante, il est nécessaire de prévoir un tube ayant un pas compris entre 20 et 40 millimètres ainsi qu'une longueur, lorsque déployée, comprise entre 80 et 120 millimètres.

Le tube hélicoïdal 8 est situé préférentiellement à une distance D du connecteur d'injection 11 reliant la fibre optique 2 à la source optique 4 comprise entre 50 centimètres et 100 centimètres. La distance D est définie comme étant la distance entre la sortie du connecteur d'injection 11 et l'extrémité le plus proche du tube hélicoïdal 8 avec le connecteur (figure 1).

La figure 4 montre une vue schématique d'une sonde 10 pour le traitement thermique endo-veineux selon un autre mode de réalisation. Cette sonde 10 reprend l'ensemble des caractéristiques de la sonde précédemment décrite. Elle présente cependant une différence au niveau de la tête d'émission 3, et plus particulièrement au niveau de la forme de celle-ci. Comme représenté sur la figure 4, la surface de d'émission 30 de la tête d'émission, qui définit la génératrice du cône, présente une forme convexe et pkus particulièrement ogivale. Cette forme permet de modifier l'angle d'émission de la sonde. A noter que le sommet de la forme en ogive correspond à celui de la forme en cône avec une génératrice droite. Cette dernière est représentée sur la figure 4 en tirets. De même que précédemment, la forme en ogive sera choisie avec un angle au sommet α de préférence de l'ordre de 57 degrés, en référence à forme conique.

L'invention est décrite dans ce qui précède à titre d'exemple. Il est entendu que l'homme du métier est à même de réaliser différentes variantes de réalisation de l'invention sans pour autant sortir du cadre de l'invention telle que définie dans les revendications ci-dessous:

## Revendications

1. Sonde (1) comprenant une fibre optique (2) comportant, en extrémité distale (2A), une tête d'émission (3), de forme conique, pour l'émission d'un rayonnement optique, la fibre optique (2) comportant un coeur (6) en silice entourée d'une gaine optique (7) en polymère, la sonde étant **caractérisée en ce qu'**elle comprend un tube (8) cintré de façon hélicoïdale et **en ce que** la fibre optique (2) traverse le tube (8) cintré de façon hélicoïdale.

2. Sonde (1) selon la revendication 1, **caractérisée en ce que** le tube présente un pas d'enroulement compris entre 20 et 40 millimètres, et de préférence de 30 millimètres.

3. Sonde (1) selon la revendication 1 ou la revendication 2, **caractérisée en ce que** le tube présente, en état déployé, une longueur comprise entre 80 et 120 millimètres, et de préférence de 100 millimètres.

4. Sonde (1) selon l'une quelconque des revendications 1 à 3, **caractérisée en ce que** le tube présente un diamètre d'enroulement comprise entre 6 et 10 millimètres, et de préférence 8 millimètres.

5. Sonde (1) selon l'une quelconque des revendications précédentes, **caractérisée en ce que** le tube est situé à une distance d'un connecteur d'injection reliant la fibre optique (2) à une source optique (4) comprise entre 50 centimètres et 100 centimètres.

6. Sonde (1) selon l'une quelconque des revendications précédentes, **caractérisée en ce que** la tête d'émission présente une forme conique avec un angle au sommet compris entre 55 et 59 degrés, et de préférence 57 degrés.

7. Sonde (1) selon la revendication précédente, **caractérisée en ce que** la tête d'émission a la forme d'une ogive ayant une génératrice courbe.

8. Sonde (1) selon l'une quelconque des revendications précédentes, **caractérisée en ce que** la tête d'émission est pourvue d'une capsule de protection (5) transparente au rayonnement optique diffusé par la tête d'émission.

9. Sonde selon l'une quelconque des revendications précédentes, **caractérisée en ce qu'**elle constitue une sonde pour le traitement endo-veineux.

## Patentansprüche

1. Sonde (1) mit einer optischen Faser (2), die am distalen Ende (2A) einen konischen Emissionskopf (3) zum Emittieren optischer Strahlung aufweist, wobei die optische Faser (2) einen Kern (6) aus Siliziumdioxid aufweist, der von einer optischen Hülle (7) aus Polymer umgeben ist, wobei die Sonde
**dadurch gekennzeichnet ist, dass** sie ein spiralförmig gebogenes Rohr (8) umfasst und **dass** die optische Faser (2) durch das spiralförmig gebogene Rohr (8) verläuft.

2. Sonde (1) nach Anspruch 1, **dadurch gekennzeichnet, dass** das Rohr eine Windungssteigung zwischen 20 und 40 Millimetern, und vorzugsweise 30 Millimetern, aufweist.

3. Sonde (1) nach Anspruch 1 oder Anspruch 2,
**dadurch gekennzeichnet, dass** das Rohr im entfalteten Zustand eine Länge zwischen 80 und 120 Millimetern, und vorzugsweise 100 Millimetern, aufweist.

4. Sonde (1) nach einem der Ansprüche 1 bis 3,
**dadurch gekennzeichnet, dass** das Rohr einen Windungsdurchmesser zwischen 6 und 10 Millimetern, und vorzugsweise 8 Millimetern, aufweist.

5. Sonde (1) nach einem der vorstehenden Ansprüche,
**dadurch gekennzeichnet, dass** sich das Rohr in einem Abstand zwischen 50 Zentimetern bis 100 Zentimetern zu einem Injektionsanschluss befindet, der die optische Faser (2) mit einer optischen Quelle (4) verbindet.

6. Sonde (1) nach einem der vorstehenden Ansprüche,
**dadurch gekennzeichnet, dass** der Emissionskopf eine konische Form mit einem Spitzenwinkel zwischen 55 und 59 Grad, und vorzugsweise 57 Grad aufweist.

7. Sonde (1) nach vorstehendem Anspruch,
**dadurch gekennzeichnet, dass** der Emissionskopf die Form einer Ogive mit gekrümmter Mantellinie aufweist.

8. Sonde (1) nach einem der vorstehenden Ansprüche,
**dadurch gekennzeichnet, dass** der Emissionskopf mit einer Schutzkapsel (5) versehen ist, die für die von dem Emissionskopf gestreute optische Strahlung transparent ist.

9. Sonde nach einem der vorstehenden Ansprüche,
**dadurch gekennzeichnet, dass** es sich um eine Sonde zur endovenösen Behandlung handelt.

## Claims

1. A probe (1) comprising an optical fiber (2) including, at its distal end (2A), a cone-shaped emission head (3) for emitting optical radiation, the optical fiber (2) having a silica core (6) surrounded by a polymer optical sheath (7), the probe being **characterized in that** it comprises a helically curved tube (8) and **in that** the optical fiber (2) passes through the helically curved tube (8).

2. The probe (1) according to claim 1, **characterized in that** the tube has a winding pitch of between 20 and 40 millimeters, and preferably 30 millimeters.

3. The probe (1) according to claim 1 or claim 2, **characterized in that** the tube, in the deployed state, has a length of between 80 and 120 millimeters, and preferably 100 millimeters.

4. The probe (1) according to any one of claims 1 to 3,
**characterized in that** the tube has a winding diameter of between 6 and 10 millimeters, and preferably 8 millimeters.

5. The probe (1) according to any one of the preceding claims,
**characterized in that** the tube is located at a distance from an injection connector connecting the optical fiber (2) to an optical source (4) of between 50 centimeters and 100 centimeters.

6. The probe (1) according to any one of the preceding claims,
**characterized in that** the emission head has a cone shape with an apical angle of between 55 and 59 degrees, and preferably 57 degrees.

7. The probe (1) according to the preceding claim, **characterized in that** the emission head has the shape of an ogive with a curved generatrix.

8. The probe (1) according to any one of the preceding claims,
**characterized in that** the emission head is provided with a protective capsule (5) that is transparent to the optical radiation scattered by the emission head.

9. The probe according to any one of the preceding claims,
**characterized in that** it constitutes a probe for endovenous treatment.
